# EUROPEAN PATENT APPLICATION

(11) **EP 0 585 235 A1**
(43) Date of publication of application: **09.03.1994**
(21) Application number: 92905650.5
(22) Date of filing: 27.12.1991
(51) Int. Cl.: C07D 249/12

(54) **METHOD FOR PRODUCING 3-NITRO-1,2,4-TRIAZOL-5-ONE (NTO)**

(71) Applicant: UNION ESPANOLA DE EXPLOSIVOS S.A., E-28006 Madrid (ES)
(72) Inventor: CILLER CORTES, Juan, Antonio, E-28027 Madrid (ES); MENDEZ PEREZ, Almudena, E-48012 Bilbao (ES)
(74) Representative: Garcia Cabrerizo, Francisco
(86) International application number: ES9100094
(87) International publication number: WO9313080

(57) **Abstract**

Method for producing 3-nitro-1,2,4-triazol-5-one (NTE), an explosive which is not very sensitive, obtained from semicarbazide (free base) and formic acid to yield formylsemicarbazide. This compound, after heating in formic acid, produces 1,2,4-triazol-3-one which leads to 3-nitro-1,2,4-triazole-5-one by reaction with nitric acid.

## Description

### BACKGROUND OF THE INVENTION

This invention relates to a method of manufacturing 3-nitro-1,2,4-triazol-5-one (NTO). NTO is an explosive which presents reduced sensitivity to impact and shock as described in US Patent 4733610. The substitution of all or part of the common explosives, such as RDX, HMX and TNT for NTO in the explosive compositions leads to a decrease in vulnerability as described in the French Patent 2584 066.

The compositions containing NTO are therefore safer and with a high level of insensibility, resistant to accidental and sympathetic initiation.

The preparation of NTO is described by G.I. Chipen, R.P. Bokalder and V.Ya. Grinshtein in Khim. Getero. Soed. 2, 110 (1966).

The raw materials used in this synthesis are semicarbazide hydrochloride and formic acid to yield the intermediate, 1,2,4-triazol-3-one which is then nitrated with nitric acid.

The first step of this process yields, together with the triazolone, substantial quantities of hydrochloric acid, according to the reaction. 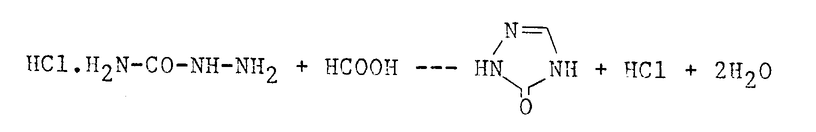
Thus each kilogram of semicarbazide hydrochloride produces 0,32 Kg. of hydrogen chloride. This is a highly toxic and corrosive gas which need to be properly treated. Metalic parts of the equipments suffers the attack of this gas, which can put them out of service after a few batches.

Accordingly, it is an object of the present invention a method to manufacture NTO, in which emission of hydrogen chloride as a byproduct of the reaction is avoided.

Another object of the present invention is to provide a method to manufacture NTO from inexpensive, starting materials.

Another object of the present invention is to provide a procedure to manufacture NTO, in standar equipment usually employed in the nitration processes for the manufacture of high explosives.

### DETAILED DESCRIPTION OF THE INVENTION

Our invention includes a procedure for the NTO manufacture starting from semicarbazide free base, according to scheme 1. 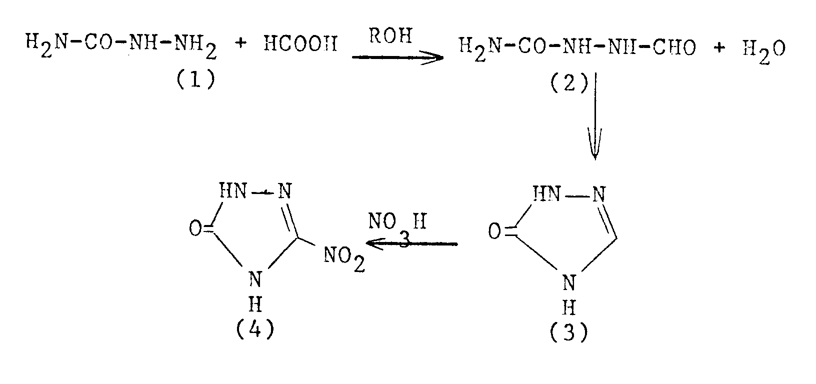
The semicarbazide used for this synthesis can be prepared from a modification of the procedure reported by T. Curtius and K. Heidenreich in Ber. Deuth. Chem. Ges 27. 55 (1894) (Prep. Org. Chem Ed. John Wiley & Sons 1972).

Reaction of semicarbazide (1) with formic acid in an appropriate solvent yields formilsemicarbazide (2). The solvent used is an aliphatic alcohol of low molecular weigth, being ethanol the preferred one. Reaction takes place at reflux temperature and the molar ratio semicarbazide/formic acid can vary between 1:1 to 1:10, being 1:4 the preferred.

After reaction is completed the flask is cooled to 15-20ºC. Formilsemicarbazide crystallizes and is filtered off. Increase yield can be obtained distilling off the excess of formic acid and ethanol.

Formilsemicarbazide (2) is then cyclized to 1,2,4 triazol-3-one (3) by means of heating in an appropriate solvent, such as formic acid. Cyclization can also be carried out without solvent, but in this case yields in triazolone are lower.

Finally nitration of triazolone with 98% nitric acid leads to 3-nitro- 1,2,4-triazol-5-one (4).

The following examples can illustrate, but in no way limit the scope of the present invention.
1. Preparation of Semicarbazide
   A mixture of 30 g of 100% Hydrazine Hydrate with 30,1 g of urea is heated at 110ºC for 4 hours. After cooling, 100 ml of absolute ethanol are added with stirring.
   After standing overnight a white solid is filtered off and washed with absolute ethanol. This product melts at 94ºC and after recrystallization with methyl or ethyl alcohol the melting point reaches 96-97ºC.
2. Preparation of triazolone
   Example 1.
   A mixture of 47 g of semicarbazide (1) and 115 g of 98% formic acid and 200 ml of ethanol is refluxed for 2 hours and then the excess of formic acid and the ethanol are distilled off . 58 g of a white solid crystallized which is characterized as formilsemicarbazide (2) m.p. 129-130º). It is dissolved in 150 ml of formic acid and refluxed for 2 hours. The excess of formic acid is distilled off. 25 g of a white solid are obtained which the most part is 1,2,4 triazol-3-one (3). This product is nitrated without further purification.
   Example 2.
   A mixture of 20 g of semicarbazide (1), 12,3 g of formic acid and 50 ml of ethanol is refluxed for 4 hours and after cooling 13,88 g of a solid crystallizes, and it is filtered off. This solid is characterized as formilsemicarbazide (2). It is dissolved in 35 ml of formic acid and refluxed for 2 hours. The excess of formic acid is distilled off. 6 g of a white solid are obtained. This product is nitrated without further purification.
   Example 3.
   10 g of formilsemicarbazide (2), prepared as described in example 1, is heated to 150º during one hour. After cooling 4 g of a white product containing triazolone is isolated and is nitrated without further purification.
3. Nitration
   In a flask containing 32 ml of 98% nitric acid inmersed in a water bath at 10ºC, 25 g of the aformentioned triazolone is added portionwise during 30 minutes. After the addition is completed the
   mixture is heated to 45ºC.
   Nitrous fumes are evolved. Then after cooling 32 ml of water are added. Cristallization occurs and the solid is filtered off and washed with cold water.
   16,25 g of NTO are isolated upon drying. The product was identified by the infrared spectroscopy, ¹³C-NMR and elemental analysis.
   Differential scanning calorimetry shows a descomposition peak at 276ºC.

## Claims

1. A method for manufacturing 3-nitro-1,2,4-triazol-5-one which comprises the following steps.
a) Reaction of semicarbazide free base with formic acid to yield formilsemicarbazide.
b) Cyclization of the formilsemicarbazide to 1,2,4-triazol-3-one by means of heating or refluxing in an appropiate solvent.
c) Nitration of 1,2,4-triazol-3-one to yield 3-nitro-1,2,4-triazol-5-one.

2. A method according to claim 1) in which the semicarbazide free base and formic acid reacts in molar ratios 1:1 to 1:10, being 1:4 the preferred.

3. A method according to claim 1) in which the semicarbazide and formic acid react in refluxing alcohol. Methyl, Ethyl, Propyl or Buthyl alcohol can be used, being Ethanol the preferred one.

4. A method according to claim 1) in which the formilsemicarbazide is isolated by means of filtration after cooling the reaction mixture at room temperature or lower if possible. The formilsemicarbazide can also be isolated after distillation of the excess of formic acid and ethanol.

5. A method according to claim 1) in which the formilsemicarbazide is cyclized to 1,2,4-triazol-3-one by heating at temperatures ranging from 130-180ºC, being 150ºC the preferred cyclization temperature.

6. A method according to claim 1) in which the formilsemicarbazide is cyclized to 1,2,4-triazol-3-one by heating in an appropriate solvent at temperatures ranging from 90 to 150ºC.

7. A method according to claim 6) in which cyclization takes place in formic acid or other high boiling hycrocarbon solvents, being formic acid the preferred one.

8. A method according to claim 1) in which the 1,2,4 triazol-3-one obtained as described in claims 5, 6 and 7 is nitrated with 98% nitric acid at temperatures ranging from 15 to 45ºC.
